# EUROPEAN PATENT APPLICATION

(11) **EP 2 667 178 A2**
(43) Date of publication of application: **27.11.2013**
(21) Application number: 13168601.6
(22) Date of filing: 21.05.2013
(51) Int. Cl.: G01N 15/14, G01N 21/45, G01N 33/543

(54) **Holographic imaging for analyzing molecules**

(30) Priority: 21.05.2012 US 201261649485 P
(71) Applicant: IMEC, 3001 Leuven (BE); VIB, 9052 Gent (BE); Universiteit Antwerpen, 2000 Antwerpen (BE); Katholieke Universiteit Leuven, 3000 Leuven (BE)
(72) Inventor: Colle, Frederik, 3001 Leuven (BE); Lagae, Liesbet, 3001 Leuven (BE); Vercruysse, Dries, 3001 Leuven (BE)
(74) Representative: Hertoghe, Kris Angèle Louisa

(57) **Abstract**

The present invention provides a device (100) for analyzing target molecules. The device comprises a substrate retention device for holding a substrate (3) having a functionalized surface (10) provided thereon. This functionalized surface is adapted for capturing a plurality of target molecules. The device also comprises a light source (1) for illuminating the functionalized surface and an image sensor (4) for recording interference patterns of magnetic particles (2) present on the functionalized surface (10). The device further comprises a first magnetic field generator (6) configured to generate a magnetic field to attract magnetic particles (2) linked to the target molecules to the functionalized surface (10). The invention further relates to a method (200) for analyzing target molecules.

## Description

### Field of the invention

The present invention relates to the field of analyzing molecules. More specifically it relates to a method and device for analyzing molecules using holographic imaging.

### Background of the invention

Magnetic particles are often employed in biological experiments as a means for separating molecules, such as for instance proteins or nucleic acids, from a complex sample. At the same time these particles can be used as labels, amplifying the binding event in the domain where the sensor is operating. The fact that magnetic particles can be used for both manipulation and detection makes them a suitable candidate for use in point of care diagnostic devices.

The use of magnetic particles does not only offer the possibility of isolating molecules from a complex matrix, but also holds the promise of decreasing the detection limit and total assay time. The limit of detection is decreased by incubating the sample with a solution of functionalized magnetic particles (nearly in-solution binding kinetics) and subsequently attracting the particles with captured molecules to a functionalized surface (increasing concentration). The externally generated magnetic field gradients are designed such that they increase the force on the particles in the direction of the surface, thereby overcoming the sedimentation limited speed.

The detection of these magnetic particles can be achieved by using sensors that measure the magnetic stray fields exhibited by these particles when they are magnetized, for example using giant magneto resistance sensors, spin-valves or magnetic tunnel junctions. These sensors are known to achieve a good sensitivity, but at the same time they can be rather expensive. This can be attributed to the need for non-standard post-processing of magnetic materials on top of the standard CMOS processed wafers.

Direct optical detection of bound magnetic particles is known, at least theoretically, to be feasible. But the use of conventional optics for counting particles requires the use of expensive optical components and a narrow field of view. This approach also results in bulky instrumentation, which may not be suited for point of care diagnostic devices.

Therefore, there exists a need for inexpensive devices which can be used for counting particles, that are limited in terms of optical component complexity and cost, and that may be manufactured using standard processing techniques.

### Summary of the invention

It is an object of embodiments of the present invention to provide good and efficient means and methods for analyzing molecules.

The above objective is accomplished by a method and device according to embodiments of the present invention.

It is an advantage of embodiments of the present invention that good time to measurement is achieved.

It is an advantage of embodiments of the present invention that an inexpensive and small readout system is provided.

It is an advantage of embodiments of the present invention that a simple substrate may be used, e.g. a simple and cheap disposable substrate for single use.

In a first aspect, the present invention relates to a device for analysing a molecule, e.g. a target molecule. The device comprises a substrate retention device for holding a substrate having a functionalized surface provided thereon. The substrate may form, but does not need to form, part of the device according to embodiments of the present invention. The functionalized surface of the substrate is adapted for capturing a plurality of target molecules. The device may comprise a functionalized surface atop the substrate configured to capture a plurality of target molecules. The device further comprises a light source for illuminating the functionalized surface, for example, a light source located above the functionalized surface and arranged so as to illuminate the functionalized surface. The device also comprises an image sensor for recording interference patterns of magnetic patterns present on the functionalized surface. For example, the device may comprise an image sensor located below the functionalized surface and arranged to record interference patterns of magnetic particles present on the functionalized surface. The device also comprises a first magnetic field generator, e.g. a first magnetic field generator located near the functionalized surface, configured to generate a magnetic field to attract magnetic particles which are linked to the target molecules to the functionalized surface. The first magnetic field generator may advantageously reduce time to measurement as the speed of the sedimentation of the target molecules to the functionalized surface is increased.

It is an advantage of embodiments of the present invention that a magnetic washing procedure may be performed, such that a microfluidics-based wash can be avoided. It is a further advantage of embodiments that it is possible to keep the overall system simple, that costs can be reduced in terms of a smaller system and that the use of reagents can be reduced.

In an embodiment of the first aspect, the functionalized surface may comprise at least one reference pattern. As an advantage, the reference pattern may assist in providing an estimate of distance between the functionalized surface and the image sensor. This information can be advantageous for an image reconstruction algorithm to perform an adequate reconstruction.

In an embodiment, the reference pattern may be a metal figure which is not radially symmetrical. The reference pattern may be, for example, a number or a letter. The correct reconstruction depth may for example be determined by performing identification on the pattern, e.g. as long as the reconstruction depth is not correct, the pattern is not identified. However, once the right reconstruction depth is found, the figure can be identified.

In an embodiment of the first aspect, the functionalized surface may comprise a plurality of regions, each region being functionalized for a different type of target molecule. As an advantage, multiple types of target molecules can be analyzed by a single functionalized surface instead of one type of target molecules.

In an embodiment of the first aspect, the substrate may comprise or may be a micro-fluidic channel, e.g. the substrate may form at least part of the micro-fluidic channel, e.g. at least one wall of the microfluidic channel. As an advantage, a solution can be transported through the micro-fluidic channel towards and away from the functionalized surface.

A substrate of a device according to embodiments of the present invention may furthermore comprise a plurality of transparent microfluidic channels, each channel comprising a functionalized surface configured to capture a plurality of target molecules. Such device according to embodiments of the present invention may correspondingly further comprise an inlet for distributing fluid to the plurality of micro-fluidic channels and an outlet arranged to drain waste fluid from the plurality of micro-fluidic channels.

In a device according to embodiments of the present invention, the image sensor may be divided into regions whereby each region records an interference pattern of a different microfluidic channel.

In an embodiment of the first aspect, a magnetic layer may further be provided for enhancing the generated magnetic field. For instance, the substrate may comprise such magnetic layer. As an additional advantage, the generated magnetic field can be enhanced passively while relaxing requirements for the magnetic field generator.

In an embodiment, the first magnetic field generator may be a toroid magnet. As an advantage, the optical path is not disrupted by the presence of such magnetic field generator.

In an embodiment of the first aspect, the device may further comprise an image processing unit configured to perform image reconstruction on the recorded interference patterns. The image processing unit may be external to the device or may be integrated in the image sensor, e.g. to provide an lab-on-chip solution in which image processing and/or reconstruction can be performed on the device itself.

In an embodiment of the first aspect, the device may further comprise a second magnetic field generator configured to generate a second magnetic field for repelling magnetic particles away from the functionalized surface. As an additional advantage, unbound particles can be washed away from the functionalized surface, such that a high sensitivity may be achieved.

In an embodiment of the first aspect, the light source may be a partially coherent light source such as a LASER.

In an embodiment of the first aspect, the device may further comprise a pin-hole for collimating the light source. As an advantage, the use of a pin-hole may relax the coherence requirements of the light source. In an embodiment, the light source may be a partially coherent light source, e.g. a LED used in combination with a pin-hole.

In particular embodiments, the present invention relates to a device for analysing target molecules, which comprises: a plurality of transparent microfluidic channels, each channel comprising a functionalized surface configured to capture a plurality of target molecules; an inlet arranged to provide fluid to the plurality of micro-fluidic channels; a light source located above the plurality of microfluidic channels arranged to illuminate the functionalized surface; an image sensor located below the plurality of microfluidic channels, arranged to record interference patterns of magnetic particles linked to the functionalized surface via a target molecule; a first magnetic field generator located near the functionalized surface and configured to generate a magnetic field to attract magnetic particles linked to the target molecules to the functionalized surface; a second magnetic field generator configured to generate a second magnetic field for repelling magnetic particles away from the functionalized surface; and an outlet arranged to exit waste fluid from the plurality of micro-fluidic channels.

In a device according to embodiments of the present invention, the image sensor may be divided into regions whereby each region records an interference pattern of a different microfluidic channel.

In a further aspect, the invention relates to a method for analysing molecules. This method may be performed using a device according to embodiments of the first aspect of the invention. A method according to this aspect of the present invention comprises providing a solution comprising a plurality of target molecules, each of the target molecules being linked to a magnetic particle. The method further comprises magnetically attracting the plurality of magnetic particles towards at least one functionalized surface. The method also comprises capturing the target molecules with the at least one functionalized surface. The method further comprises illuminating the at least one functionalized surface with an at least partially coherent light source, e.g. with a partially coherent light source. The method also comprises recording an interference pattern with an image sensor.

In a method according to embodiments of the present invention, prior to providing the solution, the preparation of the solution may be done by mixing a plurality of functionalized magnetic particles for binding to target molecules with a plurality of target molecules.

A method according to embodiments of the present invention may further comprise analysing the interference pattern for determining the target molecule concentration. The analysis may be done on or off-chip whereby an image processing unit performs image reconstruction using the recorded interference pattern.

A method according to embodiments of the present invention may further comprise repelling the plurality of magnetic particles away from the functionalized surface. As an advantage, unbound particles can be washed away before illuminating the at least one functionalized surface thereby increasing sensitivity of the device.

Particular and preferred aspects of the invention are set out in the accompanying independent and dependent claims. Features from the dependent claims may be combined with features of the independent claims and with features of other dependent claims as appropriate and not merely as explicitly set out in the claims.

These and other aspects of the invention will be apparent from and elucidated with reference to the embodiment(s) described hereinafter.

### Brief description of the drawings

FIG. 1 illustrates a device in which a light source is used to illuminate particles and an interference pattern is recorded using an image sensor, in accordance with embodiments of the present invention.
FIG. 2 illustrates means for creating a magnetic field for transporting particles towards and/or away from the surface in accordance with embodiments of the present invention.
FIG. 3 illustrates a plurality of micro-fluidic channels with functionalized surface for binding different particles in accordance with embodiments of the present invention.
FIG. 4 illustrates a micro-fluidic channel comprising a plurality of functionalized surfaces for binding particles in accordance with embodiments of the present invention.
FIG. 5 illustrates an exemplary method according to embodiments of the present invention.

The drawings are only schematic and are non-limiting. In the drawings, the size of some of the elements may be exaggerated and not drawn on scale for illustrative purposes.

Any reference signs in the claims shall not be construed as limiting the scope.

In the different drawings, the same reference signs refer to the same or analogous elements.

### Detailed description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

Devices and methods are disclosed herein for providing a small size and low cost of devices for measuring concentrations of molecules. By using holographic imaging techniques for detecting magnetic particles in accordance with the present invention, devices may be small and manufacturing costs may be low. Traditional systems known in the art, on the other hand, may use expensive and bulky optics, which may increase size and cost.

To replace the optics in traditional systems, holographic lens-free imaging techniques may be used to eliminate the magnification needed in traditional systems. Instead, an interference pattern is recorded originating from the diffraction of light around magnetic particles interfering with a reference light source. This holographic approach may be more suited for miniaturization as expensive optical components may be effectively exchanged for cheap elements, for example combined with a higher computational power requirement, e.g. for reconstructing the recorded hologram. As a result, only a light source, a substrate comprising a functionalized surface and an image sensor may be required to analyze the particles.

Where in embodiments of the present invention reference is made to "molecules" or "target molecules", reference is made to any type of molecule that is amenable to detection with the particles described herein. Particular exemplary molecules are biomolecules, for example molecules present in a biological sample, such as in a bodily fluid (blood, urine, saliva, etc.). Samples may have been pretreated to put them in a more suitable form for analysis. Biomolecules may for example include, but are not limited to, nucleic acids such as DNA or RNA, proteins, sugars and the like.

Where in embodiments reference is made to "magnetic attraction" and "magnetic repulsion", this is to be understood as merely referring to causing movement, by magnetic means, of magnetic particles in relation to a functionalized surface. This however does not imply a specific magnetic field polarity as such. For example, "magnetic repulsion" may refer to an attractive force being exerted from a source located at the side of the functionalized surface of the substrate, such that magnetic particles thereon may be pulled away from this surface, but may equally refer to a repulsive force being exerted from a source located on the side opposite of the functionalized surface, such that magnetic particles on the functionalized surface are pushed away from this surface.

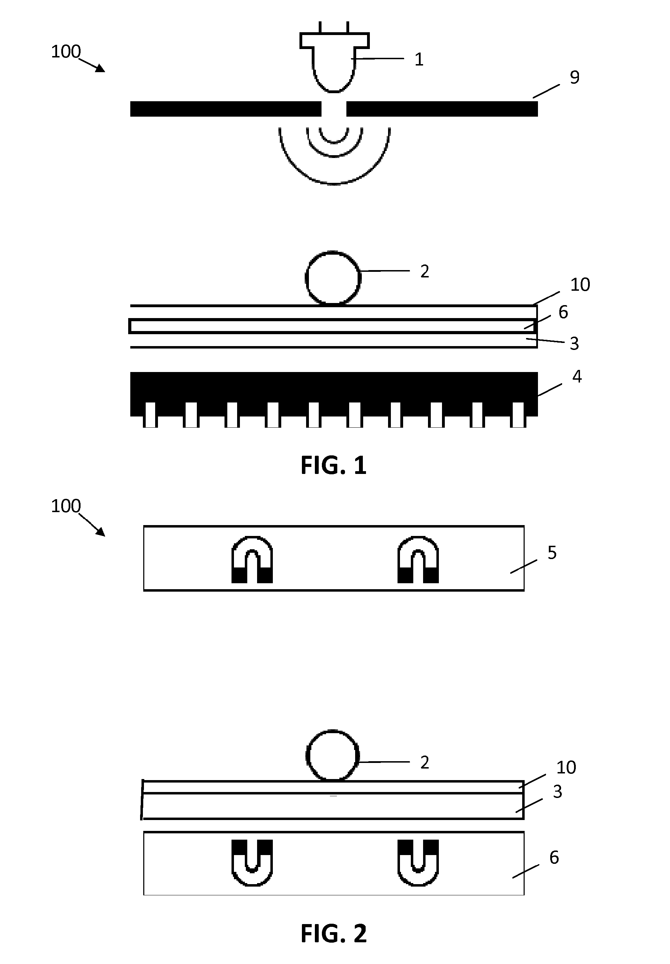

In a first aspect, the present invention relates to a device for analysing one or more molecules, e.g. a target molecules. The device comprises a substrate retention device for holding a substrate having a functionalized surface provided thereon. The substrate itself is included in the device illustrated in FIG. 1. The functionalized surface is adapted for capturing a plurality of target molecules. The device may comprise a functionalized surface atop the substrate configured to capture a plurality of target molecules. The device further comprises a light source for illuminating the functionalized surface, for example, a light source located above the functionalized surface and arranged such as to illuminate the functionalized surface. The device also comprises an image sensor for recording interference patterns of magnetic patterns present on the functionalized surface. For example, the device may comprise an image sensor located below the functionalized surface and arranged to record interference patterns of magnetic particles present on the functionalized surface. The device also comprises a first magnetic field generator, e.g. a first magnetic field generator located near the functionalized surface, configured to generate a magnetic field to attract magnetic particles which are linked to the target molecules to the functionalized surface. In an embodiment, the magnetic field gradient generator may be located near the sensor surface whereby the sensor surface is defined as a functionalized surface of the substrate.

The interference patterns, recorded by the image sensor, may be formed by interference of the diffracted light from magnetic particles with a reference light beam, e.g. emitted by the light source. In an embodiment of the first aspect, the light source may be an at least partially coherent light source such as a LASER. The device may further comprise a pin-hole for collimating the light source. As an advantage, the use of a pin-hole relaxes the coherence requirements of the light source. In an embodiment, the light source is a partially coherent light source, such as e.g. a LED which is used in combination with a pin-hole. On the detection side, an image sensor with a relatively small pixel pitch may be preferred to attain efficient use of physical space.

The substrate may be transparent, e.g. composed of quartz or glass. The functionalized surface can be made from the same material as the substrate or from some other material. The functionalized surface may preferably also be transparent. The functionalized surface may comprise gold, e.g. a gold coating applied to the substrate. This may advantageously allow the attachment of biomolecules through thiol-chemistry. Alternatively, attachment on glass can be done through silane-chemistry. However, the invention is not limited thereto, as other suitable substrates and chemistries are also known in the art.

In embodiments of the present invention, magnetic particles may be functionalized with molecules complementary to the target molecules, or with molecules having an affinity for the target molecules. The functionalized particles may be added to a complex sample comprising target molecules whereby the molecules of interest will bind to them. These complexes of magnetic particles and target molecules may be attracted by means of magnetic field gradients towards at least one functionalized surface where they will bind with the surface.

When the target molecules are nucleic acids, the magnetic particle typically may be functionalized with probes that are at least partially complementary to the molecule of interest. When the target molecules are proteins, the particles may be functionalized with at least one antibody that has an affinity for the protein of interest. Likewise, when the target molecules are sugars, the particle may be functionalized with at least one antibody or lectin that has an affinity for the molecule of interest.

FIG. 1 illustrates a device 100 according to embodiments of the present invention for analyzing target molecules. The device comprises a light source 1, which may be used for illuminating magnetic particles 2 that are bound to a functionalized surface 10 of a substrate 3. The diffraction pattern of the magnetic particles 2 can be recorded by an image sensor 4 that is located underneath the substrate 3. A pin-hole 9 may be used to create partially coherent light. The light source 1 and pin-hole 9 may be located above the substrate 3 for illuminating the magnetic particles 2, while the image sensor 4 may be located underneath the substrate 3 for recording the diffraction pattern of the magnetic particles 2. The device also comprises a first magnetic field generator 6 for generating a magnetic field to attract magnetic particles which are linked to the target molecules toward the functionalized surface.

The number thus obtained of particles bound to the functionalized surface may be a measure for the amount of molecules in the original complex sample, e.g. a fluid sample brought into contact with the substrate. By having the particles bind to the molecules of interest in the sample, nearly in-solution binding kinetics can be obtained. These are typically much faster than the kinetics of molecules binding to a two dimensional surface. By exerting a magnetic force on the magnetic particles towards the functionalized surface 10, the time it takes for all particles to reach the surface may be decreased, for example, from around 50 minutes to a mere 5 minutes. Magnetically pulling the particles away from the functionalized surface 10 can be described as a magnetic washing procedure. Therefore, a microfluidics-based wash may be avoided, making it possible to keep the overall system simple. Costs can be reduced in terms of a smaller system and the reduction of the use of reagents.

In an embodiment of the first aspect of the present invention (as also illustrated in FIG. 2), the device may also comprise a second magnetic field generator 5 configured to generate a second magnetic field for repelling magnetic particles away from the functionalized surface 10. As an additional advantage, unbound particles can be washed away from the functionalized surface. This increases the sensitivity of the device. For example, a magnetic force may be applied away from the functionalized surface to remove unbound particles. In an embodiment, the device may comprise a second magnetic field generator for washing away unbound particles, e.g. a magnetic field gradient generator arranged near above the sensor surface.

FIG. 2 illustrates a device according to such an embodiment of the present invention. Here, magnetic particles 2 are bound to a functionalized surface 10 of a substrate 3. A first magnetic field generator 6, e.g. for magnetic attraction, is used to transport magnetic particles 2 to the functionalized surface 10 of the substrate 3. The first magnetic field generator 6, e.g. a magnetic gradient field generator, may be located near the substrate 3. A second magnetic field generator 5, e.g. a magnetic field gradient generator for magnetic repulsion, may be used for washing away unbound magnetic particles. The second magnetic field generator 5 may be located near the substrate 3.

The manipulation of magnetic particles, e.g. by the first magnetic gradient field generator 6, and the recording of interference patterns, e.g. by the image sensor 4, can be physically separated into two different stages whereby the substrate slides through each stage. As an example, this could be implemented using a cartridge that is moved to different depths or stages in e.g. a handheld device.

The first 6 and/or second 5 magnetic field generator, e.g. the field gradient generators, may be designed such that the optical path remains free. For example, two electromagnets can be positioned left and right from the sensing area and designed to generate an upward force.

In an embodiment, the first and/or second magnetic field generator may be a toroid magnet. As an advantage, the optical path is not disrupted by the presence of the first magnetic field generator 6.

In particular embodiments, the first and second magnetic field generator may in fact be the same (set of) electromagnet(s), with reversible polarity. Hence if the description and claims relate to "first magnetic field generator" and "second magnetic field generator" these may be, but should not necessarily be interpreted as distinct physical entities.

In embodiments, partially coherent light may be used to illuminate the functionalized surface 10 with the bound magnetic particles. Diffraction of the light around these particles may thus generate an interference pattern, e.g. a hologram, on an image sensor 4. The mathematical description of this phenomenon enables an approximate reconstruction of the original particle distribution based on the recorded hologram, for example based on the mathematical formulation of the Huygens principle which results in a complicated integration. Furthermore, approximations of this basic formula which may be suitable for characterizing the particle distribution are known in the art, for example Fresnel approximation, Angular Spectrum formulation or Free Space Response. Given that the particle density is not too high and thus sufficient spacing is available, it may be possible to resolve the signature of individual particles. Preferably, the number of particles used is restricted in order to avoid saturation. However, if the particle density is too high, the saturation state can be determined based on signal intensity.

On the detection side, an image sensor with a relatively small pixel pitch may be preferred to attain efficient use of physical space. In embodiments of the present invention, larger pixel sizes may be compensated by adjusting the vertical distance between substrate 3 and image sensor 4. Both elements together make up the basis of the readout system, which is not only inexpensive but can also be designed to be very small.

The substrate 3 may be a glass slide or may be fabricated from another transparent material which can be functionalized. In some embodiments, the glass slide may feature some patterned reference structures. For reconstruction of the holographic image, an accurate estimate of the distance between the substrate 3 and the image sensor 4 may be provided to the mathematical algorithm. This distance may for example be in the order of tens of micrometers. Typically a few small geometries may be patterned on the substrate, such that these structures can help in iterative estimation of the reconstruction distances.

For example, in an embodiment the functionalized surface 10 may comprise at least one reference pattern. This reference pattern can provide an estimate of distance between the functionalized surface 10 and the image sensor 4. The reference pattern may be a metal figure, e.g. a figure which is not radially symmetrical. The reference pattern may for example be a number or a letter symbol. To determine the correct reconstruction depth, identification may be performed on the pattern. As long as the reconstruction depth is not correct, the pattern is not identified. Once the right reconstruction depth is found, the figure can be identified.

The simplicity of the substrate may be an advantageous feature, considering that anything besides the readout system is typically disposable and therefore should be as cheap as possible in production. In some embodiments, the magnetic field gradients may be generated outside the substrate. In particular embodiments, the magnetic field gradients may be generated within the detection system.

In some embodiments, a small magnetic layer may be used to locally enhance the magnetic fields generated by the first and/or second magnetic field generator, e.g. to locally enhance the gradient magnetic fields, e.g. the device 100, e.g. the substrate 3, may further comprise a magnetic layer for enhancing the generated magnetic field. As an additional advantage, the generated magnetic field can thus be enhanced passively while relaxing requirements for the magnetic field generator.

The force on a magnetic particle is proportional to the gradient of the magnetic field it feels. The gradient of the magnetic field is roughly proportional to the size of the structures that generate it. Addition of smaller passive magnetic structures locally may generate higher magnetic field gradients and thus larger forces than what would be produced with the external field generators alone. As magnetic materials are not fully transparent to light, addition of these structures can distort the hologram, e.g. the diffraction pattern recorded by the image sensor 4. However, this may be compensated by image processing of the hologram before reconstruction.

In an embodiment of the first aspect, the device further comprises an image processing unit configured to perform image reconstruction on the recorded interference patterns of magnetic particles. The image processing unit can be external from the device or integrated with the image sensor 4 to provide an lab-on-chip solution whereby image processing/reconstruction is performed on the device itself.

Reconstruction of the recorded hologram may require only moderate computational power, such that these calculations can for example be performed on general purpose handheld devices such as smart phones or tablets.

In an embodiment of the present invention, simultaneous detection of multiple molecular targets may be achieved by exposing the sample to a mixture of magnetic particles functionalized with different capture molecules. The complexes of magnetic particles and target molecules can be attracted towards a surface which is functionalized with multiple capture molecules in a location dependant manner.

In an embodiment, the system may be equipped with multiple micro fluidic channels in order to obtain multiplexing. Each micro-fluidic channel 7 may be functionalized with different capture molecules. This way, each of the channels can provide the means to detect a single one of the molecular targets. In both cases the location and the count of bound particles can be used to estimate the amount and the type of molecules present in the original sample.

In an embodiment of the first aspect, the substrate 3 may be a micro-fluidic channel 7. For example, the substrate 3 may form at least one wall of a micro-fluidic channel formed in or on the substrate. As an advantage, a liquid solution can be transported through the micro-fluidic channel 7 towards and away from the functionalized surface 10.

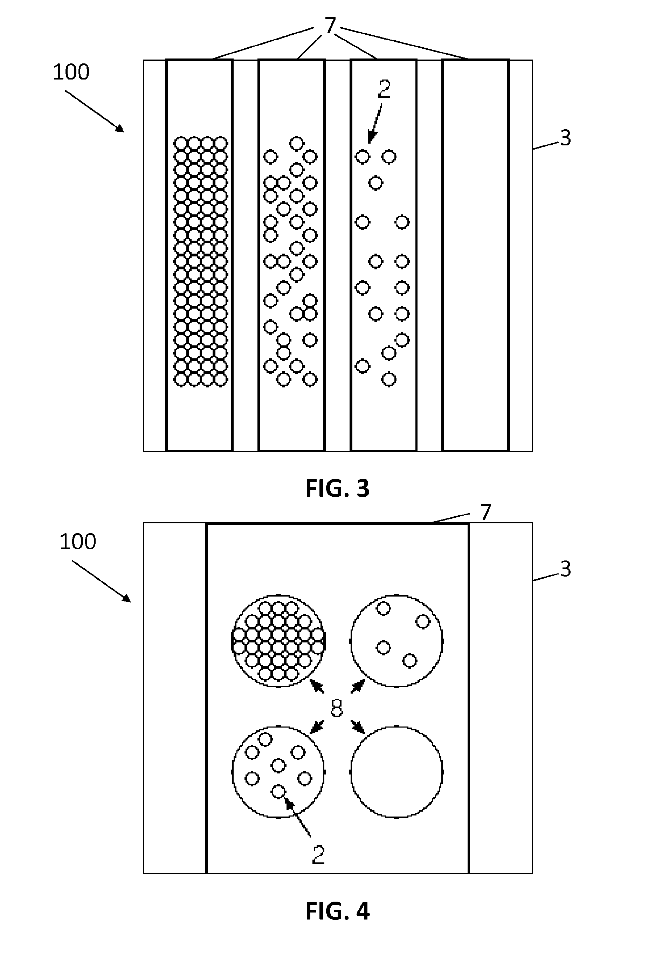

FIG. 3 illustrates another embodiment of the present invention. Here, the device 100 comprises a substrate 3. The substrate 3 may comprise a plurality of micro-fluidic channels 7, e.g. in the embodiment illustrated four micro-fluidic channels 7. Each micro-fluidic channel 7 may be functionalized for binding to a target molecule linked to a magnetic particle 2. Particularly, each micro-fluidic channel 7 may be functionalized for binding to a different target molecule.

In an embodiment of the invention, the functionalized surface 10 may comprise a plurality of regions 8, each region being functionalized for a different type of target molecule. As an advantage, multiple types of target molecules can be analysed by a single functionalized surface 10 instead of one type of target molecules.

FIG. 4 illustrates another embodiment of the present invention, in which the substrate 3 comprises a micro-fluidic channel 7. The microfluidic channel 7 may comprise a plurality of functionalized surfaces, e.g. in the embodiment illustrated four functionalized surfaces, for example functionalized spots 8. Each of the multiple functionalized surfaces may be functionalized for binding to a different target molecule. The number of functionalized surfaces can be increased or decreased depending on the target application.

In an embodiment, each micro-fluidic channel 7 may comprise a waste outlet for washing away unbound particles. The unbound particles may be washed away to prevent distorting the interference pattern of the bound particles on the functionalized surface.

For example, a device 100 according to embodiments of the present invention may comprise a plurality of transparent microfluidic channels 7, each channel comprising a functionalized surface 10 configured to capture a plurality of target molecules. Such device may also comprise an inlet for distributing fluid to the plurality of micro-fluidic channels 7 and an outlet arranged to drain waste fluid from the plurality of micro-fluidic channels 7.

In a device 100 according to embodiments of the present invention, the image sensor may furthermore be divided into regions, such that each region records an interference pattern of a different micro-fluidic channel 7.

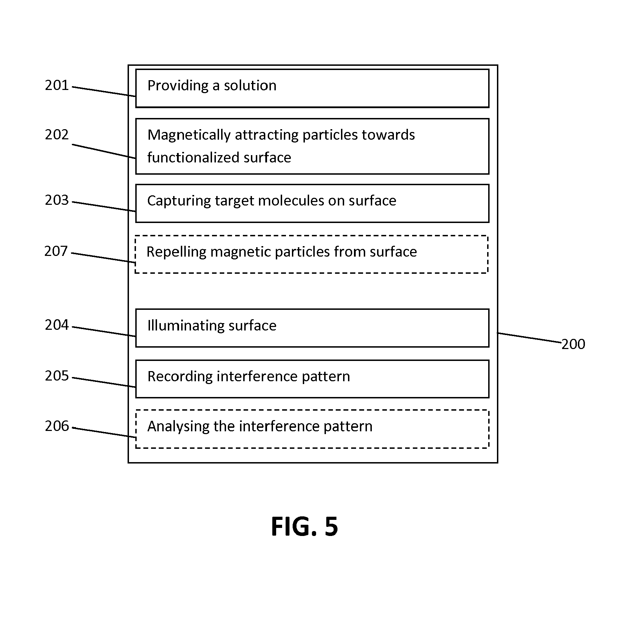

In a further aspect, the present invention also relates to a method 200 for analysing molecules, e.g. by using a device according to embodiments of the first aspect of the invention. Referring to FIG. 5, a schematic flow chart of a exemplary method 200 according to this aspect of the present invention is shown.

This method 200 comprises providing 201 a solution, e.g. an aqueous solution, which comprises a plurality of target molecules. Each of the target molecules may be linked to a magnetic particle, e.g. a representative population of all potential target molecules in the solution may be linked to a magnetic particle, e.g. substantially every target molecule in the solution may be linked to a magnetic particle. The method further comprises magnetically attracting 202 the plurality of magnetic particles towards at least one functionalized surface, e.g. using the first magnetic field generator in a device according to embodiments of the present invention. The method also comprises capturing 203 the target molecules with the at least one functionalized surface. The method also comprises illuminating 204 the at least one functionalized surface with an at least partially coherent light source, e.g. with a partially coherent light source, e.g. with a laser or a pinhole-collimated LED. The method also comprises recording 205 an interference pattern, e.g. an interference pattern generated by interference of a first portion of the light illuminating the at least one functionalized surface with a second portion thereof, the second portion being modulated by the magnetic particles on the at least one functionalized surface.

In a method 200 according to embodiments of the present invention, providing 201 the solution may comprise preparing the solution by mixing a plurality of functionalized magnetic particles for binding to target molecules with a plurality of target molecules.

The method 200 may further comprise analysing 206 the interference pattern for determining the target molecule concentration. The analysis may be done on or off-chip whereby an image processing unit performs image reconstruction using the recorded interference pattern.

A method 200 according to embodiments of the present invention may further comprise repelling 207 the plurality of magnetic particles away from the functionalized surface, e.g. using the second magnetic field generator in a device according to embodiments of the present invention. As an advantage, unbound particles can be washed away before illuminating the at least one functionalized surface thereby increasing sensitivity of the device.

## Claims

1. A device (100) for analyzing target molecules, the device (100) comprising:
- a substrate retention device for holding a substrate (3) having a functionalized surface (10) provided on said substrate (3), the functionalized surface being adapted for capturing a plurality of target molecules;
- a light source (1) for illuminating said functionalized surface;
- an image sensor (4) for recording interference patterns of magnetic particles (2) present on said functionalized surface (10); and
- a first magnetic field generator (6) configured to generate a magnetic field to attract magnetic particles (2) linked to said target molecules to said functionalized surface (10).

2. The device (100) according to claim 1, furthermore comprising a substrate (3) having a functionalized surface (10) provided on said substrate (3), the functionalized surface being adapted for capturing a plurality of target molecules.

3. The device (100) according to claim 2, wherein said functionalized surface (10) comprises at least one reference pattern configured to provide an estimate distance between said functionalized surface and said image sensor to an image reconstruction algorithm.

4. The device (100) according to any of claims 2 or 3, wherein the functionalized surface (10) comprises a plurality of regions (8), each region being functionalized for a different type of target molecule.

5. The device (100) according to any of claims 2 to 4, wherein the functionalized surface (10) comprises a gold layer.

6. The device (100) according to any of claims 2 to 5, the substrate (3) furthermore comprising:
- a plurality of transparent microfluidic channels (7), each channel comprising a functionalized surface (10) configured to capture a plurality of target molecules;
- an inlet for distributing fluid to said plurality of micro-fluidic channels (7); and
- an outlet arranged to drain waste fluid from said plurality of micro-fluidic channels (7).

7. The device (100) according to any of claims 2 to 6, in which said substrate (3) forms at least part of a micro-fluidic channel (7).

8. The device (100) according to any of the previous claims, further comprising a magnetic layer for enhancing said generated magnetic field.

9. The device (100) according to any of the previous claims, further comprising an image processing unit configured to perform image reconstruction of said recorded interference patterns of magnetic particles (2).

10. The device (100) according to any of the previous claims, further comprising a second magnetic field generator (5) configured to generate a second magnetic field for repelling magnetic particles away from said functionalized surface (10).

11. The device (100) according to any of the previous claims, wherein said light source (1) is a partially coherent light source.

12. The device (100) according to any of the previous claims, further comprising a pin-hole (9) for collimating said light source (1).

13. A method (200) for analyzing molecules, the method comprising:
- providing (201) a solution comprising a plurality of target molecules, each of target molecule being linked to a magnetic particle;
- magnetically attracting (202) the plurality of magnetic particles towards a functionalized surface (10);
- capturing (203) the target molecules with the functionalized surface (10);
- illuminating (204) the functionalized surface (10) with an at least partially coherent light source (1); and
- recording (205) an interference pattern with an image sensor (4).

14. The method (200) according to claim 13, further comprising: analyzing (206) said interference pattern for determining the target molecule concentration.

15. The method (200) according to any of claims 13 or 14, further comprising:
repelling (207) the plurality of magnetic particles away from the at least one functionalized surface (10) to remove unbound particles before illuminating the at least one functionalized surface.
